Europäisches Patentamt

European Patent Office

Office européen des brevets

(19)

(11) Publication number: 0 259 105
A2

# EUROPEAN PATENT APPLICATION

(12)

(21) Application number: 87307614.5

(22) Date of filing: 27.08.87

(51) Int. Cl.⁴: C 07 C 41/06
C 07 C 43/04, B 01 J 27/053,
B 01 J 21/06

(30) Priority: 03.09.86 GB 8621263

(43) Date of publication of application:
09.03.88 Bulletin 88/10

(84) Designated Contracting States:
BE DE FR GB IT NL SE

(71) Applicant: The British Petroleum Company p.l.c.
Britannic House Moor Lane
London EC2Y 9BU (GB)

(72) Inventor: Atkins, Martin Philip
The British Petroleum Company p.l.c. Chertsey Road
Sunbury-on-Thames Middlesex, TW16 7LN (GB)

Ball, William John
The British Petroleum Company p.l.c. Chertsey Road
Sunbury-on-Thames Middlesex, TW16 7LN (GB)

Smith, David John Harry
The British Petroleum Company p.l.c. Chertsey Road
Sunbury-on-Thames Middlesex, TW16 7LN (GB)

(74) Representative: Richardson, Derek et al
BP INTERNATIONAL LIMITED Patents & Agreements
Division Chertsey Road
Sunbury-on-Thames Middlesex TW16 7LN (GB)

(54) Proton catalysed reactions.

(57) Processes for carrying out proton-catalysed reactions are carried out in the presence of a solid acidic catalyst which is a metal oxide containing surface acid groups obtainable by treating a solid metal oxide containing residual hydroxyl groups with an acid, or a mixture of metal oxides so-obtainable. Particularly useful metal oxides are zirconia and stannic oxide.

EP 0 259 105 A2

**Description**

PROTON CATALYSED REACTIONS

The present invention relates in general to reactions which are catalysed by protons and in particular to the reaction of an olefin with an alcohol to produce an ether.

Many different types of organic reaction are catalysed by protons or, to give them another name, hydrogen ions. Typical of such reactions may be mentioned the production of alcohols by the hydration of olefins, the production of alkyl aromatic hydrocarbons by reaction of an aromatic hydrocarbon with an alkylating agent the production of esters by reacting an alcohol with a carboxylic acid and many more. A proton-catalysed reaction in which there is considerable current interest is the production of ethers, for use either as individual chemicals or as gasoline blending components. Particular attention has been given to the production of methyl tertiary butyl ether (MTBE) from methanol and isobutene.

Generally the protons are provided by the dissociation of a strong mineral acid or a strong organic acid. Thus, sulphuric acid and para-toluene sulphonic acid have been used extensively in the industrial production of esters, and phosphoric acid, usually supported on silica, is a catalyst commonly employed in the commercial production of ethanol. A disadvantage associated with the use of free acid is that to separate them from the reaction product a neutralisation step is generally required, while supported free acids generally tend to leach from the support resulting in catalyst deactivation. Both free and supported acids generally give rise to corrosion problems and acid effluents. As a result there has been a move away from the use of mineral and organic acids towards the use of solid catalysts in proton-catalysed reactions.

Strongly acidic cation-exchange resins consisting essentially of a sulphonated polystyrene resin, for example a divinylbenzene crosslinked polystyrene matrix having from 0.5 to 20% and preferably from 4 to 16% of copolymerised divinylbenzene, attached to which are ionisable or functional sulphonic acid groups, have been used as catalysts in proton-catalysed reactions, in particular the production of ethers. A disadvantage of cation-exchange resins is their generally low thermal stability, which restricts their use to reactions operable at lower temperatures, for example temperatures below about 150°C.

Acidic forms of zeolites have also been used as catalysts in proton-catalysed reactions, but in certain reactions their catalytic activity is too low.

The use of cation-exchangeable layered and pillared clays as catalysts in proton-catalysed reactions is also known from our European patent publication numbers 0031252, 0031687 and 0083970 respectively.

In Polish Patent No. 123,515 there is described a novel solid catalyst for the preparation of ethers, which catalyst is prepared by saturating a gel-type carrier with an organic O-S compound containing at least one-$SO_3H$ group and at least one-$COOH$ and/or $-OH$ group followed by thermal activation at 100-250°C. As the O-S compound there may be used compounds such as phenol sulphonic acid, sulphosalicylic acid, sulphopyrocatechnic acid, phenoldisulphonic acid, naphtholdisulphonic acid or resorcinolsulphonic acid. Gel-type carriers, such as silica gel, Al, Ti, Zr, or mixed gels containing Si and/or Al, Ti, or Zr oxides are employed.

US Patent No. 4,487,976 discloses the etherification of phenols over transition metal oxides, for example W, Hf, Nb, Ta and Zr, containing $SO_4^{2-}$ anions. Specifically exemplified is the production of anisole and a small amount of cresol by passing phenol, methanol and nitrogen over tungsten oxide containing $SO_4^{2-}$ anions.

We have now found that proton-catalysed reactions, and particularly the production of ethers, are catalysed by acid-treated solid metal oxides.

Accordingly, the present invention provides a process for carrying out a proton-catalysed reaction in the presence of a solid acidic catalyst characterised in that the catalyst is a metal oxide containing surface acid groups obtainable by treating a solid metal oxide containing residual hydroxyl groups with an acid, or a mixture of metal oxides so-obtainable.

In a preferred embodiment the invention provides a process for producing a catalysed as aforesaid which process comprises the following steps

    (A) precipitating a metal compound thermally decomposable to the oxide from a solution of a soluble salt thereof,

    (B) calcining the precipitated metal compound obtained in step (A) to an extent such as to form the basic oxide structure while retaining residual hydroxyl groups, and

    (C) treating the calcined product of step (B) with an acid so as to produce surface acid groups.

The metal oxide may be chosen from oxides of the metals of Groups II, III, IV or V of the Periodic Table of the Elements. Suitable metal oxides include zirconia, stannic oxide, magnesia and vanadia. Preferred metal oxides include zirconia and stannic oxide.

The acid used may be either an inorganic or an organic acid, for example sulphuric acid, hydrochloric acid, sulphonic acid, phosphoric acid, a hydrocarbyl-sulphonic acid or a carboxylic acid. Preferable the acid is a strong inorganic acid, more preferably sulphuric acid. Alternatively, the solid metal oxide containing residual hydroxyl groups may be treated with a sulphonating agent, for example sodium hydrogen sulphite, to produce a sulphonated material.

In the aforesaid process for producing a catalyst the precursor of the metal hydroxide is produced in step (A) by precipitation from a solution of a soluble salt thereof. The solution is preferably an aqueous solution, though solutions in other solvents may be employed. Suitable salts of the metals include the chlorides, nitrates, carbonates and carboxylates. Techniques for precipitating metal compounds thermally decomposable to their oxides from solutions of their salts are well known in the art. Generally, this may be accomplished by the addition of a base, for example aqueous ammonia.

In step (B) of the process the precipitated metal compound thermally decomposable to the oxide formed in step (A) is calcined to an extent such as to form the basic oxide structure, while retaining residual hydroxyl groups. The extent of calcination is an important parameter in determining the activity of the final catalyst. Thus, experiments have shown that using zirconia, in the absence of a calcination step, i.e. using the metal hydroxide, a relatively inactive catalyst is obtained. Again, using zirconia, experiments have suggested an optimum calcination temperature of at least about 450°C. Calcination at 250°C produces a relatively inactive catalyst. It is predicated from this that the calcination must be sufficient to give predominantly oxide but that there must be sufficient hydroxyl groups remaining to combine chemically with the acid rather than to give a certain amount of loosely held acid. The optimum calcination temperature for other metal compounds thermally decomposable to the oxide will depend on the nature of the metal hydroxide and may be readily determined by experiment. Experiment has also shown that active catalysts are not generally produced when calcination is effected after acid treatment, lending further support for the importance of pre-calcination.

Provided the level of calcination is correct, the acid will combine under relatively mild conditions. Good results have been achieved with metal oxides treated with 0.5 M sulphuric acid at ambient temperature.

The processes capable of catalysis by protons include:

(i) a process for the production of an ether by reacting either an olefin or an olefin oxide with an alcohol,

(ii) a process for the production of an alkyl aromatic compound by reating an aromatic hydrocarbon with an alkylating agent selected from alcohols and olefins,

(iii) a process for the production of an alcohol by reacting an olefin with water, or

(iv) a process for the production of an ester by reacting either an olefin or an olefin oxide with a carboxylic acid.

Suitable reactants and reaction conditions for operating the processes (i) to (iv) are described in EP-A-0083970 which is incorporated by reference herein.

A preferred process comprises reacting a $C_4$ to $C_7$ monoolefin, or a mixture thereof, with an alcohol at elevated temperature to produce an ether.

Although any $C_4$ to $C_7$ monoolefin may be employed, it is preferred to use either isobutene, which may be substantially pure or may contain monoolefinic impurities, or a pentene, for example a methylbutene, which may be substantially pure or may be admixed with lower or higher monoolefins, for example butenes, including isobutenes, or hexenes.

Suitably the alcohol may be an alkanol, for example methanol, ethanol, propanols, butanols, pentanols, or hexanols, preferably methanol.

A preferred reaction is that of isobutene with methanol to produce MTBE. Another preferred reaction is that of a methylbutene with methanol to produce a tertiary amyl methyl ether (TAME). A preferred source of monoolefin reactants is the refinery stream generally referred to as Light Cat Cracked Spirit (LCCS), itself derived from the catalytic cracking of heavier petroleum fractions, normally boiling in the range from 350 to 550°C, to ligher products.

The reaction of the monoolefin with methanol may suitably be operated at a temperature in the range from 40 to 120°C and a pressure in the range from 4 to 80 bar gauge. The process may be operated either in the liquid or the vapour phase, preferably in the liquid phase, suitably using the catalyst in the form of a fixed bed.

The olefin to alcohol molar ratio may range from olefin rich to alcohol rich, e.g. a molar range from 10:1 to 1:10. Operating olefin-rich simplifies the product recovery since there can be high conversion of the alcohol and negligible azeotrope formations. However, an excess of olefin may encourage undesirable side reactions such as olefin dimerisation or oligomerisation, and to avoid this it is sometimes preferred to operate alcohol rich or to adhere strictly to the stoichiometric olefin:alcohol molar ratio.

The invention will now be further illustrated by reference to the following examples.

Example 1

Preparation of Zirconium Hydroxide

600 g of zirconium chloride were dissolved in 2400 ml of distilled water and concentrated ammonia solution was added dropwise until the pH was alkaline.

The precipitated zirconium hydroxide was filtered off, washed with distilled water, and dried in an oven at 100°C.

15 ml portions of the zirconium hydroxide produced in Example 1 were calcined and then acid treated using a number of calcination temperatures and acid treatments as follows:

3

|  | Calcination | Acid Treatment |
|---|---|---|
| Example 2 | 250°C for 4 hours | Washed in a filter funnel with 300 mls of 0.5M sulphuric acid, followed by drying at 100°C. |
| Example 3 | 450°C for 4 hours | As for Example 2 |
| Example 4 | 600°C for 4 hours | As for Example 2 |
| Example 5 | 350°C for 4 hours | As for Example 2 |
| Example 6 | 350°C for 4 hours | Refluxed with 100ml of 0.1M sulphuric acid for 4 hours, followed by drying at 100°C |

Examples 7 - 8

15 ml of the zirconium hydroxide produced in Example 1 were acid-treated and then calcined as follows:

|  | Acid Treatment | Calcination |
|---|---|---|
| CT 1 | Washed in a filter funnel with 300ml of 0.5M sulphuric acid, followed by drying at 100°C | 250°C for 4 hours |
| CT 2 | As for CT 1 | 350°C for 4 hours |

Example 9

Tin powder (23.7g) was added slowly to a stirred aqueous solution of nitric acid (300ml distilled water/100ml concentrated nitric acid) at 95-100°C. On completion of addition the slurry was stirred for 5 minutes at 95°C, cooled to room-temperature and filtered. The filter-cake was resuspended in distilled water (300ml) at 40°C and an aqueous solution of ammonia (0.910 $NH_3$ : $H_2O$ = 1:2, volume) added until the pH was 7.0. The mixture was cooled to room temperature, filtered and the filter-cake washed in distilled water (3 x 300ml) and finally dried at 75°C.

30ml of stannic oxide prepared as above were calcined at 450°C for 4 hours. After cooling, it was placed in a filter funnel and washed with 600 mls of 0.5 M sulphuric acid. It was then dried at 100°C.

Example 10

Use of Catalysts of MTBE Production

The apparatus used was a continuous flow unit having a 25 ml reactor immersed in an oil bath. The reactor contained 10 $cm^3$ of 1.5-1 mm size catalyst particles. Isobutene and methanol at a 1:1 molar ratio were passed over the catalyst at an LHSV of 4 v/v/hr. The isobutene/methanol feed was diluted with pentane (90%). The reactor was maintained at 80°C and 15 bars, giving liquid phase operation. The product from the reactor was cooled to 0°C and analysed by gas chromatography. Each run was of 8 hours duration.

Table 1 gives the MTBE production for each of the catalysts of Examples 1 to 9.

4

TABLE 1

| CATALYST | % wt MTBE formed in Product Stream | | |
| --- | --- | --- | --- |
| | At 1 hour | At 2 hours | At 8 hours |
| Example 1 | 1.0 | 1.9 | 6.0 |
| Example 2 | 2.1 | 5.7 | 6.8 |
| Example 3 | 1.9 | 12.5 | 12.9 |
| Example 4 | 3.0 | 12.1 | 13.1 |
| Example 5 | 1.5 | 2.0 | 2.0 |
| Example 6 | 1.1 | 1.5 | 3.8 |
| CT 1 | 2.8 | 2.1 | 1.2 |
| CT 2 | 1.0 | 1.5 | 2.0 |
| Example 7 | 3.5 | 9.1 | 13.0 |

The Table shows that the dried zirconium hydroxide (Example 1) had a limited activity for MTBE production. Calcination and acid treatment (Examples 2 - 5) increased the activity, with a particularly good result after calcination at 450°C and 600°C (Examples 3 and 4).

If acid treatment preceded calcination (Comp Tests 1 and 2), the catalysts produced had negligible activity.

Finally Example 7 shows that a calcined and acid treated stannic oxide catalyst had comparable activity to the calcined and acid treated zirconia catalysts.

**Claims**

1 A process for carrying out a proton-catalysed reaction in the presence of a solid acidic catalyst characterised in that the catalyst is a metal oxide containing surface acid groups obtainable by treating a solid metal oxide containing residual hydroxyl groups with an acid, or a mixture of metal oxides so-obtainable.

2 A process for producing a metal oxide containing surface acid groups for use as catalyst in the process of claim 1 which process comprises the following steps:-

(A) precipitating a metal compound thermally decomposable to the oxide from a solution of a soluble salt thereof,

(B) calcining the precipitated metal compound obtained in step (A) to an extent such as to form the basic oxide structure while retaining residual hydroxyl groups, and

(C) treating the calcined product of step (B) with an acid so as to produce surface acid groups.

3 A process according to either claim 1 or claim 2 wherein the metal oxide is zirconia.

4 A process according to either claim 1 or claim 2 wherein the metal oxide is stannic oxide.

5 A process according to any one of the preceding claims wherein the acid is sulphuric acid.

6 A process according to claim 2 wherein the metal oxide is zirconia and the calcination temperature is at least about 450°C.

7 A process according to any one of claims 1 and 3 to 6 wherein a $C_4$ to $C_7$ monoolefin, or a mixture thereof, is reacted with an alcohol to produce an ether.

8 A process according to claim 7 wherein isobutene is reacted with methanol to produce methyl tertiary butyl ether.

9 A process according to claim 7 wherein methylbutene is reacted with methanol to produce a tertiary amyl methyl ether.

10 A process according to claim 7 wherein the source of the monoolefin reactant is the refinery stream identified as Light Cat Cracked Spirit.